# EUROPEAN PATENT APPLICATION

(11) **EP 4 585 583 A1**
(43) Date of publication of application: **16.07.2025**
(21) Application number: 24862980.0
(22) Date of filing: 26.06.2024
(51) Int. Cl.: C07C 29/76, C07C 29/80, C07C 29/04, C07C 31/10

(54) **METHOD OF PREPARING ISOPROPYL ALCOHOL**

(30) Priority: 08.09.2023 KR 20230119554
(71) Applicant: LG CHEM, LTD., Seoul 07336 (KR)
(72) Inventor: HWANG, Sung June, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2024/008883
(87) International publication number: WO 2025/053397

(57) **Abstract**

The present invention provides a method of preparing isopropyl alcohol (IPA), and the method may include (S1) feeding a reaction product of a propylene monomer and water to a gas purification unit and separating gas components including an unreacted propylene monomer; (S2) feeding the reaction product from which the gas components are separated to a first organic material removal tower, and discharging an upper stream containing organic by-products and isopropyl alcohol and a lower stream containing isopropyl alcohol, an n-propyl alcohol (NPA) by-product, and water; (S3) feeding the upper discharge stream of the first organic material removal tower to a second organic material removal tower, introducing process circulating water, and discharging an upper stream containing organic by-products and a lower stream containing isopropyl alcohol and process circulating water; and (S4) feeding the lower discharge stream of the first organic material removal tower to an isopropyl alcohol (IPA) purification unit including a plurality of distillation towers and separating isopropyl alcohol.

## Description

### [Technical Field]

### CROSS-REFERENCE TO RELATED APPLICATION

The present application claims the benefit of priority to Korean Patent Application No. 10-2023-0119554, filed on September 8, 2023, the entire contents of which are incorporated herein by reference.

### Technical Field

The present invention relates to a method of preparing isopropyl alcohol, and more particularly, to a method for reducing the amount of energy used and minimizing a loss in isopropyl alcohol.

### [Background Art]

Isopropyl alcohol (IPA) is used for various purposes including a solvent for a cleaning agent, a raw material for an industrial paint or reagent, a paint, an ink, or the like in the electronics industry such as the manufacture of a semiconductor, a liquid crystal display (LCD), or the like.

Such an isopropyl alcohol may be prepared by reacting propylene with water. For example, in a reaction unit, a propylene monomer and water are subjected to a vapor phase reaction to obtain a reaction product containing isopropyl alcohol, an unreacted propylene monomer, unreacted water, and by-products such as organic materials, and the vapor phase reaction product is subjected to a process of passing through a downstream process tower, separating a propylene monomer, water, and by-products, and purifying isopropyl alcohol.

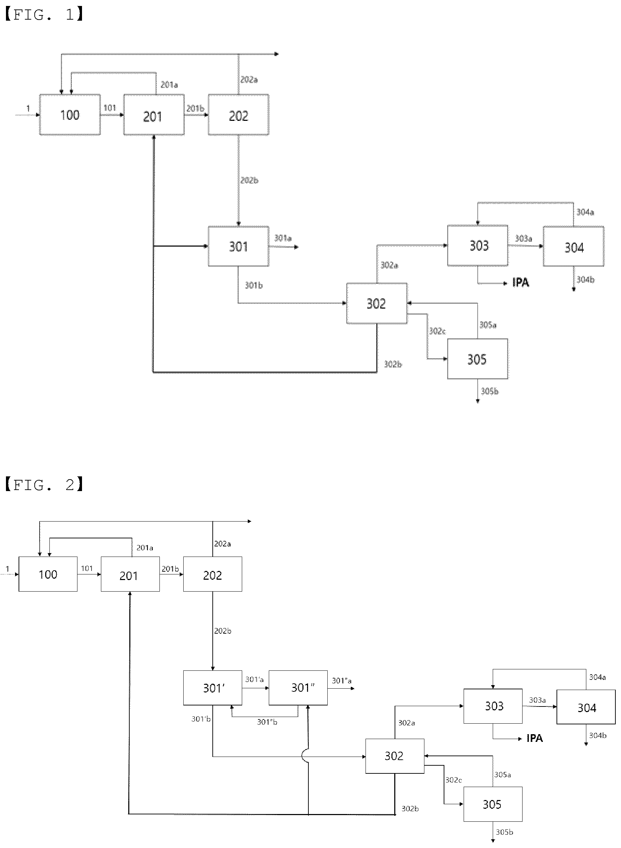

According to an IPA preparation process as illustrated in FIG. 1, a feed 1 containing a propylene monomer and water is fed to a reactor 100 and a reaction is allowed to proceed to obtain a vapor phase reaction product 101, unreacted propylene gas 201a and low boiling point gas components 202a are removed from the reaction product 101 in a gas purification unit including an absorption tower 201 and a gas purification tower 202, a stream 202b containing IPA, water, and by-products, from which the gas components are removed, is fed to an organic material removal tower 301 to remove organic materials, an upper stream 302a containing IPA, a lower stream 302b containing water, and a side stream 302c containing NPA are separated in a water removal tower 302, and the upper stream 302a is fed to an IPA recovery tower 303, thereby obtaining IPA from which water is separated by a solvent. A stream 303a containing a solvent and water, which is separated from the IPA recovery tower 303, is separated into an upper stream 304a of a solvent and a lower stream 304b of water in a solvent recovery tower 304.

In the IPA purification process, a part of water 302b recovered through a lower portion of the water removal tower 302 may be recovered and used as process circulating water. Typically, eight times more circulating water than the amount of IPA produced is used, about 40% of the circulating water used is introduced into the absorption tower 201 so that vapor phase IPA may be absorbed into water and then separated in a liquid phase, and the remaining, which is about 60% of the original amount, is introduced into the organic material removal tower 301 and used to remove organic by-products such as diisopropyl ether (DIPE) and hexene using liquid-liquid phase equilibrium.

In this case, as the liquid phase stream 202b containing IPA, water, and by-products, from which the gas components are removed in the upstream gas purification unit, and a large amount of circulating water are introduced together into the organic material removal tower 301, a feed flow rate of the water removal tower 302 disposed downstream of the organic material removal tower 301 may increase excessively, resulting in an increase in steam demand.

Therefore, there is a demand for a method for realizing energy savings by reducing steam demand throughout the IPA preparation process.

### [Disclosure]

### [Technical Problem]

In order to solve the problems mentioned in the background art, an object of the present invention is to provide a method for minimizing increases in energy consumption and cost by reducing the amount of circulating water used in an isopropyl alcohol preparation process.

### [Technical Solution]

In one general aspect, a method of preparing isopropyl alcohol includes:
(S1) feeding a reaction product of a propylene monomer and water to a gas purification unit and separating gas components including an unreacted propylene monomer;
(S2) feeding the reaction product from which the gas components are separated to a first organic material removal tower, and discharging an upper stream containing organic by-products and isopropyl alcohol and a lower stream containing isopropyl alcohol (IPA), an n-propyl alcohol (NPA) by-product, and water;
(S3) feeding the upper discharge stream of the first organic material removal tower to a second organic material removal tower, introducing process circulating water, and discharging an upper stream containing organic by-products and a lower stream containing isopropyl alcohol and process circulating water; and
(S4) feeding the lower discharge stream of the first organic material removal tower to an isopropyl alcohol (IPA) purification unit including a plurality of distillation towers and separating isopropyl alcohol.

### [Advantageous Effects]

According to the present invention, two organic material removal towers are applied, in the first organic material removal tower, an upper stream containing organic by-products and a part of isopropyl alcohol is separated without feeding process circulating water, and in the second organic material removal tower, the separated upper stream is brought into contact with a small amount of process circulating water to dissolve IPA so as to separate organic by-products and isopropyl alcohol, such that a feed flow rate of a downstream IPA purification unit is lowered, thereby reducing the amount of steam used.

The reduction in the amount of steam used in the IPA purification unit may realize energy savings in the entire process and may reduce the size of the distillation tower used for IPA purification, thereby minimizing cost increases.

### [Description of Drawings]

FIG. 1 illustrates a conventional IPA preparation process procedure.
FIG. 2 illustrates a method of preparing IPA according to an embodiment of the present invention.

### [Best Mode]

The terms and words used in the description and claims of the present invention are not to be construed limitedly as having general or dictionary meanings but are to be construed as having meanings and concepts meeting the technical idea of the present invention, based on a principle that the inventors are able to appropriately define the concepts of terms in order to describe their own inventions in the best mode.

The meaning "including" or "containing" used in the present application concretely specifies a specific property, region, integer, step, operation, element, and/or component, and does not exclude addition of another specific property, region, integer, step, operation, element, and/or component.

The term "stream" used in the present application may refer to a flow of a fluid in a process, and may also refer to a fluid flowing through a pipe itself. Specifically, the stream may refer to both a fluid flowing through a pipe connecting respective devices to each other itself and a flow of a fluid. In addition, the fluid may include any one or more components of gas, liquid, and solid.

The term "upper portion" used in the present application refers to a point at a height of 0 to 20% from the uppermost portion of a device to a lower portion, and specifically, may refer to the top (of the tower), unless otherwise specified. In addition, the term "lower portion" refers to a point at a height of 80 to 100% from the uppermost portion of the device to the lower portion, and specifically, may refer to the bottom (of the tower).

The term "side stream" used in the present application may refer to a stream discharged at a height of 25 to 80% or 40 to 70% from the uppermost portion of the device to the lower portion.

In addition, the "pressure" referred to in the present application refers to gauge pressure measured based on atmospheric pressure.

Meanwhile, in the present invention, in devices such as an absorption tower, a purification tower, a removal tower, and a recovery tower, an operating temperature of the device may refer to a temperature of an upper portion or a lower portion of the device, unless otherwise specified. In addition, an operating pressure of the device may refer to a pressure of the upper portion of the device, unless otherwise specified.

An embodiment of the present invention relates to a method of preparing isopropyl alcohol (IPA). Hereinafter, the method of preparing IPA of the present invention will be described in detail with reference to the drawings.

FIG. 2 illustrates the method of preparing isopropyl alcohol according to an embodiment of the present invention. Isopropyl alcohol may be prepared using a system including a reactor 100; a gas purification unit including an absorption tower 201 and a gas purification tower 202; a first organic material removal tower 301' and a second organic material removal tower 302''; and an IPA purification unit including a water removal tower 302, an IPA recovery tower 303, a solvent recovery tower 304, and a high boiling point material removal tower 305. In addition, the system may be used by additionally installing a reboiler for transferring heat to a feed stream of each tower and heating the feed stream, a condenser that converts an upper stream produced by the heating from a vapor phase to a liquid phase, a decanter for liquid-liquid separation of the condensed stream, a valve that controls flow of streams, a pump, and the like.

Referring to FIG. 2, a feed 1 containing a propylene monomer and water may be fed to the reactor 100, and a reaction product 101 containing isopropyl alcohol may be obtained through a vapor phase reaction process.

Since only a part of the propylene monomer fed to the reactor 100 is used in the reaction, the reaction product may contain an unreacted propylene monomer and unreacted water in addition to isopropyl alcohol produced by the reaction of the propylene monomer and water. For example, the reaction product 101 may contain 65 to 85 wt% of an unreacted propylene monomer, 4 to 8 wt% of isopropyl alcohol, and 5 to 30 wt% of water. In addition, the reaction product may contain organic materials such as isopropyl ether (DIPE), hexene, hexanol, and acetone, n-propyl alcohol (NPA), and the like, as by-products. Therefore, there is a need for performing a process of separating unreacted raw materials from the reaction product and purifying isopropyl alcohol from various by-products.

First, the reaction product 101 obtained by the vapor phase reaction of the propylene monomer and water is fed to the gas purification unit, and gas components including an unreacted propylene monomer are separated (S1).

Specifically, the reaction product 101 is fed through a lower portion of the absorption tower 201, process circulating water is introduced through an upper portion of the absorption tower 201, and the unreacted propylene monomer is separated. In this case, as the process circulating water, water recovered in the distillation tower included in the downstream IPA purification unit, that is, the water removal tower 302, may be used. In the absorption tower 201, vapor phase isopropyl alcohol contained in the reaction product may be absorbed into the process circulating water and then obtained through a lower liquid phase stream 201b, and a vapor phase stream 201a containing an unreacted propylene monomer may be separated through the upper portion. The unreacted propylene monomer contained in the vapor phase stream 201a is recovered to the reactor 100 and reused as a raw material for preparing isopropyl alcohol.

In an embodiment of the present invention, a flow rate of the process circulating water fed to the absorption tower 201 may be 15 to 40 wt% or 15 to 35 wt% of a flow rate of the reaction product. When the process washing water is supplied at a flow rate within the above range, absorption capacity of isopropyl alcohol contained in the reaction product may be improved, and at the same time, the energy cost for recovery of the process circulating water in the downstream process may be prevented from increasing excessively.

The absorption tower 201 may be operated at a temperature of 90 to 100°C or 90 to 95°C and a pressure of 25 to 40 kg/cm²·g or 25 to 35 kg/cm²·g. When the above operating conditions are satisfied, the upper discharge stream containing an unreacted propylene monomer and the lower discharge stream containing isopropyl alcohol may be effectively separated.

Meanwhile, the lower liquid phase stream of the absorption tower 201 may contain a small amount of low boiling point gas components including an unreacted propylene monomer, which are not separated, in addition to isopropyl alcohol and unreacted water. For example, a content of the low boiling point gas components including an unreacted propylene monomer contained in the lower liquid phase stream of the absorption tower 201 may be 5 wt% or less or 2 to 5 wt%.

Accordingly, the liquid phase stream 201b containing isopropyl alcohol separated from the absorption tower 201 may be fed to one or more gas purification towers 202, and may be separated into an upper stream 202a containing low boiling point components and a lower stream 202b containing isopropyl alcohol, water, and by-products.

If necessary, the lower liquid phase stream of the absorption tower 201 may be fed to the gas purification tower 202 after passing through one or more flash drums. That is, first, in the flash drum, the low boiling point components may be primarily separated and then fed to the gas purification tower 202 again, an unreacted propylene monomer and low boiling point components of inert gases may be separated through an upper portion of the gas purification tower 202, and a trace amount of high boiling point components may be separated through a lower portion of the gas purification tower 202.

The upper stream 202a containing low boiling point components separated from the gas purification tower 202 may contain an unreacted propylene monomer and an inert gas (for example, ethane or propane). The unreacted propylene monomer contained in the upper stream of the gas purification tower 202 may be recovered to the reactor 100, and the inert gas may be branched off for exhaust.

Meanwhile, the lower stream 202b separated from the gas purification tower 202 may contain isopropyl alcohol, water, and organic materials such as n-propyl alcohol (NPA), isopropyl ether (DIPE), hexene, and acetone as by-products.

Therefore, it is required to remove the organic by-products from the lower discharge stream 202b of the gas purification tower.

In the conventional IPA preparation process, in order to remove the organic by-products from the reaction product from which the gas components are separated, the process circulating water recovered in the water removal tower 302 was fed to the organic material removal tower and used to separate the organic by-products using liquid-liquid phase equilibrium. The process circulating water was fed in an amount of 70 to 95 wt% or 80 to 90 wt% of a flow rate of the feed stream of the organic material removal tower and used to dissolve isopropyl alcohol. In this case, since the process circulating water used in the upstream adsorption tower and the process circulating water used in the organic material removal tower are combined and introduced into the distillation tower of the downstream IPA purification unit, there is a problem of an increase in steam demand due to an excessive flow rate.

In order to solve these problems, in the present invention, two organic material removal towers are applied, and in the first organic material removal tower, the upper stream containing organic by-products and a part of isopropyl alcohol is separated without feeding process circulating water, and in the second organic material removal tower, the separated upper stream is brought into contact with a small amount of process circulating water to dissolve IPA so as to remove organic by-products.

To be more specific, first, the reaction product from which the gas components are separated, that is, the lower discharge stream 202b of the gas purification tower, is fed to the first organic material removal tower 301', and separation of components according to boiling point is performed without feeding process circulating water (S2).

The stream fed to the first organic material removal tower 301' may contain isopropyl alcohol, water, an n-propyl alcohol (NPA) by-product, and organic by-products (for example, diisopropyl ether (DIPE), hexene, hexanol, acetone, or a mixture thereof).

Since process circulating water is not fed during the component separation in the first organic material removal tower 301', dissolution of isopropyl alcohol by water does not occur, and thus, a part of isopropyl alcohol may be contained, together with the organic by-products, in the stream that is heated by heat provided in the reboiler connected to a lower portion of the first organic material removal tower 301', moves to an upper portion of the first organic material removal tower 301', passes through the condenser, and is then discharged. For example, an upper stream 301'a of the first organic material removal tower may be contained in an amount of 5 to 25 wt%, and specifically 10 to 20 wt%, of the entire isopropyl alcohol fed to the first organic material removal tower.

As a result, the stream fed to the first organic material removal tower 301' may be separated into the upper stream 301'a containing organic by-products and isopropyl alcohol, and a lower stream 301'b containing isopropyl alcohol, an n-propyl alcohol (NPA) by-product, and water.

In order to recover the isopropyl alcohol contained in the upper stream 301'a of the first organic material removal tower, the upper discharge stream 301'b of the first organic material removal tower is fed to the second organic material removal tower 301'', a small amount of process circulating water is introduced into the second organic material removal tower 301'', and an upper stream 301''a containing organic by-products and a lower stream 301''b containing isopropyl alcohol and process circulating water are separated (S3).

More specifically, the second organic material removal tower 301'' is disposed downstream of the condenser connected to the first organic material removal tower 301', and the stream 301'a containing organic by-products and isopropyl alcohol is fed to the second organic material removal tower 301''. In this case, a content of the isopropyl alcohol contained in the stream 301'a fed to the second organic material removal tower 301'' is equivalent to 10% to 20% of a content of the isopropyl alcohol contained in the feed stream of the first organic material removal tower 301'. Therefore, even when a small amount of process circulating water is introduced into the second organic material removal tower 301", the isopropyl alcohol contained in the feed stream 301'a may be effectively separated. That is, the isopropyl alcohol contained in the feed stream is selectively dissolved using a difference in solubility by a small amount of process circulating water, and then moves to the lower portion, such that the isopropyl alcohol may be separated from the organic by-products.

The isopropyl alcohol dissolved in the process circulating water is discharged through the lower stream 301"b of the second organic material removal tower and then recycled to the first organic material removal tower 301', which may minimize a loss in isopropyl alcohol.

In an embodiment of the present invention, the first organic material removal tower 301' may be operated at a temperature of 75 to 130°C or 80 to 105°C and a pressure of 0 to 2 kg/cm²·g or 0 to 0.5 kg/cm²·g. When the above operating conditions are satisfied, the organic by-products may be effectively separated from the mixed stream without feeding process circulating water.

In addition, the second organic material removal tower 301'' may be operated at a temperature of 75 to 125°C or 80 to 105°C and a pressure of 0 to 2 kg/cm²·g or 0 to 0.5 kg/cm²·g. When the above operating conditions are satisfied, the efficiency of dissolving isopropyl alcohol may be increased even if a small amount of process circulating water is introduced.

In the second organic material removal tower 301'', a flow rate of the process circulating water may be adjusted so that the process circulating water and isopropyl alcohol come into contact with each other at a weight ratio of 2.5:1 to 5:1. Such a flow rate of the process circulating water is significantly reduced compared to the process circulating water fed in an amount of about 80 wt% or more with respect to the amount of the feed stream in the existing organic material removal tower in the conventional IPA preparation process. This may lower a feed flow rate of the distillation tower included in the downstream IPA purification unit, which may reduce the amount of steam used.

Meanwhile, the liquid phase stream 301'b separated through the lower portion of the first organic material removal tower 301' may contain, based on the total weight of the liquid phase stream 301'b, 9 to 12 wt% of isopropyl alcohol, 0.1 to 1 wt% of n-propyl alcohol, and 87 to 90 wt% of water.

That is, the liquid phase stream 301'b may contain a large amount of water including unreacted water discharged from the reactor 100 and the process circulating water used to absorb vapor phase isopropyl alcohol in the absorption tower 201, and n-propyl alcohol (NPA), which is a reaction by-product, and therefore, in the IPA purification unit including a plurality of distillation towers, a large amount of water and the NPA by-product are removed to separate high purity isopropyl (S4).

Specifically, the lower discharge stream 301'b of the first organic material removal tower 301' is fed to the water removal tower 302, and an upper stream 302a containing isopropyl alcohol, a lower stream 302b containing water, and a side stream containing n-propyl alcohol (NPA) are separated and discharged.

The upper stream 302a separated from the water removal tower 302 may contain an azeotrope of isopropyl alcohol and water, and for example, may contain 85 to 90 wt% of isopropyl alcohol and 10 to 15 wt% of water.

The water separated through the lower stream 302b of the water removal tower 302 may be recovered and used as process circulating water. As described above, the water separated from the water removal tower 302 may be fed to the upstream absorption tower 201 and used to absorb vapor phase isopropyl alcohol. In addition, the water separated from the water removal tower 302 may be used for heat exchange for preheating of water fed to the reactor 100, if necessary, because it has a high temperature exceeding 100°C.

The water removal tower 302 may be operated at a temperature of 70 to 150°C or 80 to 140°C and a pressure of 0 to 5 kg/cm²·g or 0 to 3 kg/cm²·g. When the above operating conditions are satisfied, the azeotrope of isopropyl alcohol and water may be effectively separated.

Thereafter, the upper discharge stream 302a of the water removal tower 302 may be fed to the IPA recovery tower 303, an organic solvent (for example cyclohexane, benzene, or the like) may be added as an azeotropic agent, and an upper stream 303a containing water and an organic solvent and a lower stream containing IPA may be discharged. That is, in the IPA recovery tower 303, the azeotrope of isopropyl alcohol and water is broken due to the organic solvent, such that isopropyl alcohol purified with high purity may be obtained.

The stream 303a containing water and an organic solvent, which is separated through an upper portion of the IPA recovery tower, may be fed to the solvent recovery tower 304, and an upper stream 304a of a solvent and a lower stream 304b of water may be separated and discharged. The solvent stream 304 may be recycled to the IPA recovery tower 303.

Meanwhile, a side discharge stream 302c of the water removal tower 302 containing n-propyl alcohol (NPA) may be fed to the high boiling point material removal tower 305, a stream 305b containing NPA may be discharged through a lower portion of the high boiling point material removal tower 305, and an upper stream 305a may be recycled to the water removal tower 302.

As described above, according to the present invention, two organic material removal towers are applied, in the first organic material removal tower, the upper stream containing organic by-products and a part of isopropyl alcohol is separated without feeding process circulating water, and in the second organic material removal tower, the separated upper stream is brought into contact with a small amount of process circulating water to dissolve IPA so as to separate organic by-products and isopropyl alcohol, such that a feed flow rate of the downstream IPA purification unit is lowered, thereby reducing the amount of steam used.

The reduction in the amount of steam used in the IPA purification unit may realize energy savings in the entire process and may reduce the size of the distillation tower used for IPA purification, thereby minimizing cost increases.

### [Mode for Invention]

Hereinafter, the present invention will be described in more detail with reference to Examples. However, the following Examples are provided for illustrating the present invention. It is apparent to those skilled in the art that various modifications and alterations may be made without departing from the scope and spirit of the present invention, and the scope of the present invention is not limited thereto.

In the following Examples and Comparative Examples, the method according to the present invention was simulated using the commercial process simulation program Aspen Plus. As the constants required for the simulation, the values stored in the program, the values described in the literature, and the like were used.

### Example 1

As illustrated in FIG. 2, isopropyl alcohol was prepared using a system including a reactor 100; a gas purification unit including an absorption tower 201 and a gas purification tower 202; a first organic material removal tower 301' and a second organic material removal tower 302''; and an IPA purification unit including a water removal tower 302, an IPA recovery tower 303, a solvent recovery tower 304, and a high boiling point material removal tower 305.

### (Step 1) Gas Separation in Gas Purification

In the reactor 100, a feed 1 containing water at 100°C and a propylene monomer was fed and subjected to a vapor phase reaction, the obtained reaction product 101 was fed through a lower portion of the absorption tower 201, and 40% of the process circulating water recovered in the water removal tower 302 of the IPA purification unit was introduced through an upper portion of the absorption tower 201.

In the absorption tower 201, a liquid phase stream 201b into which vapor phase isopropyl alcohol was absorbed was discharged through a lower portion, and a vapor phase stream 201a containing an unreacted propylene monomer was separated through an upper portion and then circulated to the reactor 100.

The liquid phase stream 201b into which isopropyl alcohol was absorbed was passed through the gas purification tower 202, and then separated into a low boiling point upper stream 202a and a lower stream 202b containing isopropyl alcohol, water, and by-products.

### (Step 2) Separation of Organic By-products without Feeding Process Circulating Water in First Organic Material Removal Tower

The lower stream 202b of the gas purification tower was fed to the first organic material removal tower 301', and separation of components according to boiling point was performed without feeding process circulating water, thereby separating the lower stream 202b into an upper stream 301'a containing organic by-products and a part of isopropyl alcohol (about 10% of the entire isopropyl alcohol) and a lower stream 301'b containing isopropyl alcohol, an n-propyl alcohol (NPA) by-product, and water.

### (Step 3) Introduction of Process Circulating Water and Recovery of Partial IPA in Second Organic Material Removal Tower

The upper discharge stream 301'b of the first organic material removal tower 301' was fed to the second organic material removal tower 301'', the process circulating water recovered in the water removal tower 302 of the IPA purification unit was partially fed to the second organic material removal tower 301'', and an upper stream 301''a containing organic by-products and a lower stream 301''b containing isopropyl alcohol and process circulating water were separated. The lower stream 301''b was recovered and recycled to the first organic material removal tower 301'. At this time, a feed flow rate of the process circulating water was controlled so that the process circulating water and isopropyl alcohol were in contact with each other at a flow rate ratio of 3:1 in the second organic material removal tower 301'', and as a result, the process circulating water was fed in an amount of 6 wt% of a flow rate of the stream 202b fed to the first organic material removal tower 301'.

### (Step 4) IPA Separation in IPA Purification Unit

The stream 301'b discharged through the lower portion of the first organic material removal tower 301' was fed to the water removal tower 302, and an upper stream 302a containing isopropyl alcohol, a lower stream 302b containing water, and a side stream containing n-propyl alcohol (NPA) were separated and discharged. Water was recovered through the lower stream 302b, and then the lower stream 302b was partially fed to the upstream absorption tower 201 and second organic material removal tower 301''.

The upper discharge stream 302a of the water removal tower 302 was fed to the IPA recovery tower 303, cyclohexane was added as an azeotropic agent, an upper stream 303a containing water and cyclohexane was discharged, and IPA was separated through the lower portion.

Meanwhile, a side discharge stream 302c of the water removal tower 302 containing n-propyl alcohol (NPA) was fed to the high boiling point material removal tower 305, a stream 305b containing NPA was discharged through a lower portion of the high boiling point material removal tower 305, and an upper stream 305a was recycled to the water removal tower 302.

### Comparative Example 1

As illustrated in FIG. 1, isopropyl alcohol was prepared using a system including a reactor 100; a gas purification unit including an absorption tower 201 and a gas purification tower 202; one organic material removal tower 301; and an IPA purification unit including a water removal tower 302, an IPA recovery tower 303, a solvent recovery tower 304, and a high boiling point material removal tower 305.

### (Step 1) Gas Separation in Gas Purification

The same process as in Step 1 of Example 1 was performed.

### (Step 2) Feeding of Process Circulating Water and Separation of Organic By-Products in One Organic Material Removal Tower

A lower stream 202b of the gas purification tower was fed to the organic material removal tower 301, the process circulating water recovered in the water removal tower 302 of the IPA purification unit was fed in an amount of 88 wt% of a flow rate of the stream 202b fed to the organic material removal tower 301, and a liquid phase 301a containing organic by-products and a liquid phase 301b containing isopropyl alcohol and water were separated. As the process circulating water, water separated and recovered in the downstream water removal tower 302 was used.

### (Step 3) IPA Separation in IPA Purification Unit

A stream 301b discharged through a lower portion of the organic material removal tower 301 was fed to the water removal tower 302, and an upper stream 302a containing isopropyl alcohol, a lower stream 302b containing water, and a side stream containing n-propyl alcohol (NPA) were separated and discharged. Water was recovered through the lower stream 302b, and then the lower stream 302b was fed to the upstream absorption tower 201 and organic material removal tower 301.

The upper discharge stream 302a of the water removal tower 302 was fed to the IPA recovery tower 303, cyclohexane was added as an azeotropic agent, an upper stream 303a containing water and cyclohexane was discharged, and IPA was separated through the lower portion.

Meanwhile, a side discharge stream 302c of the water removal tower 302 containing n-propyl alcohol (NPA) was fed to the high boiling point material removal tower 305, a stream 305b containing NPA was discharged through a lower portion of the high boiling point material removal tower 305, and an upper stream 305a was recycled to the water removal tower 302.

### Comparative Example 2

The same process as in Comparative Example 1 was performed, except that the process circulating water recovered in the water removal tower 302 of the IPA purification unit was fed in an amount of 6 wt% of the flow rate of the feed stream 202b in the organic material removal tower 301.

Table 1 shows the comparison of the IPA purification processes and results according to Example and Comparative Examples.

**[Table 1]**

| IPA purification process | | Example 1 | Comparative Example 1 | Comparative Example 2 |
|---|---|---|---|---|
| Organic material removal tower | Feed stream flow rate¹⁾ | 1 | 1 | 1 |
| | Circulating water flow rate (First tower) | - | 0.88 | 0.06 |
| | Circulating water flow rate (Second tower) | 0.06 | - | - |
| | Organic material removal rate | 100% | 100% | 100% |
| Feed stream flow rate of water removal tower | | 1. 18 | 1. 98 | 1.16 |
| IPA recovery rate | | 99.98% | 99.98% | 76% |
| Amount of steam used²⁾ | | 0.77 | 1 | 1 |
| 1) The flow rate of the stream fed to the organic material removal tower is set to "100", and based on this, the flow rates of the process circulating water and the feed stream of the water removal tower are expressed in a ratio. | | | | |
| 2) The amount of steam used indicates the total amount of steam used in the organic material removal tower (including the first and second towers) and the water removal tower. | | | | |

As can be seen in Table 1, in Example 1, in the first organic material removal tower, the upper stream containing organic by-products and a part of IPA was separated without feeding process circulating water, a small amount of process circulating water (6% of the flow rate of the feed stream of the first organic material removal tower) was fed to the second organic material removal tower to dissolve IPA contained in the upper stream of the first organic material removal tower, and organic by-products and isopropyl alcohol were separated, lowering the feed flow rate of the water removal tower in the IPA purification unit. As a result, the amount of steam used was reduced, and a high IPA recovery rate was maintained.

On the other hand, in Comparative Example 1, as the process circulating water equivalent to 88% of the flow rate of the feed stream was introduced into one organic material removal tower, the flow rate of the feed stream of the water removal tower increased excessively, resulting in an increase in the amount of steam used.

Meanwhile, in Comparative Example 2, in which a different flow rate of circulating water was applied under the same conditions as the amount of steam used in Comparative Example 1, as a small amount of process circulating water was introduced while applying one organic material removal tower, a feed flow rate of the water removal tower was lowered, but the amount of circulating water was insufficient compared to that of IPA in the feed stream in the organic material removal tower, and IPA was not sufficiently dissolved. As a result, it was confirmed that an IPA recovery rate was significantly reduced.

### [Detailed Description of Main Elements]

- 100:: Reactor
- 201:: Absorption tower
- 202:: Gas purification tower
- 301, 301', 301'':: Organic material removal tower
- 302:: Water removal tower
- 303:: IPA recovery tower
- 304:: Solvent recovery tower
- 305:: High boiling point material removal tower

## Claims

1. A method of preparing isopropyl alcohol, the method comprising:
(S1) feeding a reaction product of a propylene monomer and water to a gas purification unit and separating gas components including an unreacted propylene monomer;
(S2) feeding the reaction product from which the gas components are separated to a first organic material removal tower, and discharging an upper stream containing organic by-products and isopropyl alcohol and a lower stream containing isopropyl alcohol (IPA), an n-propyl alcohol (NPA) by-product, and water;
(S3) feeding the upper discharge stream of the first organic material removal tower to a second organic material removal tower, introducing process circulating water, and discharging an upper stream containing organic by-products and a lower stream containing isopropyl alcohol and process circulating water; and
(S4) feeding the lower discharge stream of the first organic material removal tower to an isopropyl alcohol (IPA) purification unit including a plurality of distillation towers and separating isopropyl alcohol.

2. The method of claim 1, wherein step (S1) includes bringing the reaction product of the propylene monomer and water into contact with the process circulating water in an absorption tower of the gas purification unit to remove a vapor phase stream containing an unreacted propylene monomer, and then separating low boiling point gas components from a liquid phase stream containing components absorbed into the process circulating water.

3. The method of claim 1, wherein a stream fed to the first organic material removal tower in step (S2) contains isopropyl alcohol, water, an n-propyl alcohol (NPA) by-product, and organic by-products.

4. The method of claim 3, wherein the organic by-products include diisopropyl ether (DIPE), hexene, hexanol, acetone, or a mixture thereof.

5. The method of claim 1, wherein in step (S2), process circulating water is not fed to the first organic material removal tower.

6. The method of claim 1, wherein the upper discharge stream of the first organic material removal tower is contained in an amount of 5 to 15 wt% of the entire isopropyl alcohol fed to the first organic material removal tower.

7. The method of claim 1, wherein in step (S3), the second organic material removal tower is disposed downstream of a condenser connected to the first organic material removal tower.

8. The method of claim 1, wherein the process circulating water fed to the second organic material removal tower selectively dissolves the isopropyl alcohol in the stream containing organic by-products and isopropyl alcohol to induce liquid-liquid separation.

9. The method of claim 8, wherein the isopropyl alcohol dissolved in the process circulating water is discharged through a lower portion of the second organic material removal tower and then recycled to the first organic material removal tower.

10. The method of claim 1, wherein in the second organic material removal tower, the process circulating water is fed at a flow rate at which the process circulating water and the isopropyl alcohol are in contact with each other at a weight ratio of 2.5:1 to 5:1.

11. The method of claim 1, wherein step (S4) includes:
feeding the lower discharge stream of the first organic material removal tower to a water removal tower of the IPA purification unit, and discharging an upper stream containing a mixture of isopropyl alcohol and water, a lower stream containing water, and a side stream containing n-propyl alcohol (NPA);
feeding the upper discharge stream of the water removal tower and an organic solvent to an IPA recovery tower of the IPA purification unit, and discharging an upper stream containing water and an organic solvent and a lower stream containing IPA;
feeding the upper discharge stream of the IPA separation tower to a solvent recovery tower of the IPA purification unit and discharging an upper stream of a solvent and a lower stream of water; and
feeding the side discharge stream of the water removal tower to a high boiling point material removal tower of the IPA purification unit, and discharging a stream containing NPA through a lower portion of the high boiling point material removal tower.

12. The method of claim 11, wherein the water contained in the lower discharge stream of the water removal tower is recovered in the IPA purification unit and used as process circulating water.
